(19) Europäisches Patentamt
European Patent Office
Office européen des brevets

(11) **EP 4 177 904 A1**

(12) **EUROPEAN PATENT APPLICATION**

(43) Date of publication:
**10.05.2023 Bulletin 2023/19**

(21) Application number: **22205628.5**

(22) Date of filing: **04.11.2022**

(51) International Patent Classification (IPC):
**G16H 30/40** (2018.01)   **G16H 40/63** (2018.01)
**G16H 50/20** (2018.01)

(52) Cooperative Patent Classification (CPC):
**G16H 50/20; G16H 30/40; G16H 40/63**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **05.11.2021   JP 2021181131**

(71) Applicant: **Canon Medical Systems Corporation
Tochigi 324-0036 (JP)**

(72) Inventor: **NORO, Kazumasa
Tochigi, 324-0036 (JP)**

(74) Representative: **Marks & Clerk LLP
15 Fetter Lane
London EC4A 1BW (GB)**

(54) **MEDICAL INFORMATION PROCESSING APPARATUS AND METHOD**

(57)     An information processing apparatus according includes processing circuitry. The processing circuitry adds a correct answer label used for training a decision making model, which is used for decision making in a medical care field, in accordance with an operator's input instruction. The processing circuitry collects status data indicating an operator's status while doing the work of adding a correct answer label. The processing circuitry trains a reliability level determination model which accepts status data and outputs a reliability level of a correct answer label. The processing circuitry obtains input data of a decision making model. The processing circuitry trains a decision making model which accepts input data and outputs output data indicating a result of decision making.

FIG. 5

# Description

## CROSS-REFERENCE TO RELATED APPLICATION(S)

This application is based upon and claims the benefit of priority from Japanese Patent Application No. 2021-181131, filed November 5, 2021.

## FIELD

Embodiments described herein relate generally to medical information processing apparatus and method.

## BACKGROUND

Semi- or full automation of job operations using machine learning models is expected to improve the efficiency of job operations that require high-level expertise by specialists. As work criteria of such job operations are left to the judgement of a person who conducts the job operation, such job operations are called personalized job operations. In the medical field, typical examples of job personalization are jobs related to medical decision making such as image diagnosis and determination of examination protocols. In supporting personalized job operations using machine learning, it is difficult to accurately add correct answer labels because of factors such as the operator's skill and the situation at the time a correct answer label is added, and for this reason, the accuracy of prediction by a machine learning model may be deteriorated.

## SUMMARY

In relation to the embodiments, the following matters are disclosed as one aspect and a selective feature of the present invention.

[1] A medical information processing apparatus comprising processing circuitry configured to:

add a correct answer label used for training a decision making model, which is a machine learning model used for decision making in a medical care field, in accordance with an operator's input instruction;
collect status data indicating a status of the operator while doing the work of adding the correct answer label;
train, based on the status data and the correct answer label, a reliability level determination model, which is a machine learning model which accepts status data and outputs a reliability level of the correct answer label; and
obtain input data of the decision making model; and
train, based on the input data, the correct answer label, and the reliability level, the decision making model which accepts the input data and outputs output data that is data indicating a result of the decision making.

[2] The processing circuitry may collect, as the status data, data relating to operator's operations, lines of sight, speeches, and/or facial expressions that reflect a process of an operator's decision making at the time of doing the work.

[3] The processing circuitry may collect, as data relating to the line of sight, reference item data, which is data relating to an item that the line of sight of the operator focuses on among various items displayed on a display screen for the work of addition.

[4] The processing circuitry may collect, as the reference item data, an identifier of a reference item on which the operator's line of sight focuses.

[5] The processing circuitry may collect ability data, which is data relating to an ability of the operator, and train the reliability level determination model which accepts the status data and the ability data, and outputs the reliability level based on the status data, the ability data, and the correct answer label.

[6] The processing circuitry may further collect additional data, which is data relating to a freshness level, a confidence level, a quality, and/or a required time, and train the reliability level determination model which accepts the status data, the ability data, and the additional data and outputs the reliability level based on the status data, the ability data, the additional data, and the correct answer label.

[7] The reliability level determination model may be a multi-class classification model that outputs the probability of each of multiple classes relating to a result of the decision making as the reliability level.

[8] The processing circuitry may train the decision making model by minimizing a loss function.
The loss function may include an error between an output of the decision making model and the correct answer label weighted by the reliability level.

[9] The processing circuitry may display the reliability level via a display device.

[10] The decision making may be an addition of annotation of a disease candidate area to a medical image.
The processing circuitry may display the annotation with a color value according to the reliability level.

[11] The correspondence between the reliability level and the color value may be set in accordance with a difficulty level of the addition of the annotation.

[12] A medical information processing method comprising:

adding a correct answer label used for training a decision making model, which is a machine learning model used for decision making in a medical care field, in accordance with an operator's input instruction;

collecting status data indicating a status of the operator while doing the work of adding the correct answer label;

training, based on the status data and the correct answer label, a reliability level determination model, which is a machine learning model which accepts status data and outputs a reliability level of the correct answer label;

obtaining input data of the decision making model; and

training, based on the input data, the correct answer label, and the reliability level, the decision making model which accepts the input data and outputs output data that is data indicating a result of the decision making.

BRIEF DESCRIPTION OF THE DRAWINGS

FIG. 1 is a diagram showing a configuration example of a medical information system according to the present embodiment.

FIG. 2 is a diagram showing a configuration example of a medical information processing apparatus shown in FIG. 1.

FIG. 3 is a diagram showing a relationship between an input and an output of a decision making model in a learning phase and an operation phase.

FIG. 4 is a diagram showing a relationship between an input and an output of a reliability level determination model in a learning phase and an operation phase.

FIG. 5 is a diagram showing an example of the flow of medical information processing by the medical information processing apparatus.

FIG. 6 is a diagram schematically showing adding of a correct answer label and collection of status data in step S3 of FIG. 5.

FIG. 7 is a diagram showing a relationship between an input and an output of a reliability level determination model in a learning phase.

FIG. 8 is a diagram showing an example of training data.

FIG. 9 is a diagram showing a relationship between an input and an output of a reliability level determination model in an operation phase.

FIG. 10 is a diagram showing an example of a reliability database.

FIG. 11 is a diagram showing an example of input data.

FIG. 12 is a diagram showing a relationship between an input and an output of an examination protocol classification model in a learning phase.

FIG. 13 is a diagram showing a relationship between an input and an output of a trained examination protocol classification model in an operation phase.

FIG. 14 is a diagram showing a relationship between an input and an output of a reliability level determination model according to Modification 1 in a learning phase.

FIG. 15 is a diagram showing an example of training data according to Modification 1.

FIG. 16 is a diagram showing a relationship between an input and an output of a reliability level determination model according to Modification 2 in a learning phase.

FIG. 17 is a diagram showing an example of a display screen of a reliability level according to Modification 3.

FIG. 18 is a diagram showing an example of a correspondence between frequency distribution of a reliability level and a color value.

FIG. 19 is a diagram showing another example of a correspondence between frequency distribution of a reliability level and a color value.

DETAILED DESCRIPTION

A medical information processing apparatus according to the embodiment has a processing circuitry. The processing circuitry adds a correct answer label used for training a decision making model, which is a machine learning model used for decision making in the medical field, in accordance with an operator's input instruction. The processing circuitry collects status data indicating a status of the operator while doing the work of adding the correct answer label. The processing circuitry trains a reliability level determination model, which is a machine learning model which accepts status data and outputs a reliability level of the correct answer label, based on the status data and the correct answer label. The processing circuitry obtains input data of the decision making model. The processing circuitry trains the decision making model which accepts the input data and outputs output data that is data indicating a result of the decision making, based on the input data, the correct answer label, and the reliability level.

An embodiment of the medical information processing apparatus and method will be described in detail below with reference to the accompanying drawings.

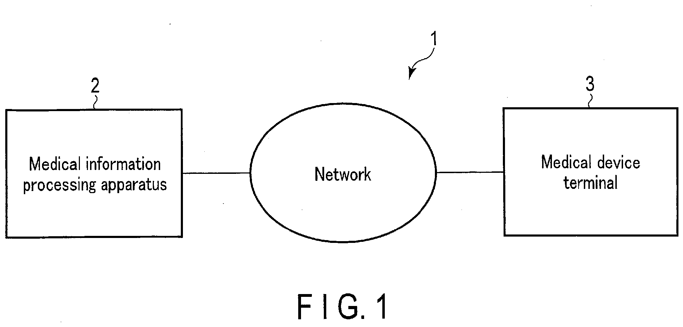

FIG. 1 is a diagram showing a configuration example of a medical information processing system 1 according to the present embodiment. As shown in FIG. 1, the medical information processing system 1 is a computer system having a medical information processing apparatus 2 and a medical device terminal 3. The medical information processing apparatus 2 and the medical device terminal 3 are connected to each other by wire or wirelessly in a communicable manner. The medical information processing apparatus 2 is an information processing terminal such as a computer of, for example, a workstation processing medical information. The medical device terminal 3 is an information processing terminal capable of transmitting operator's instructions to the medical information processing apparatus and having a processor, a storage apparatus, an input device, a communication device, and a display device. As the medical device terminal 3, an installation-type computer, a laptop computer, a tablet device, a smartphone, etc., can be adopted.

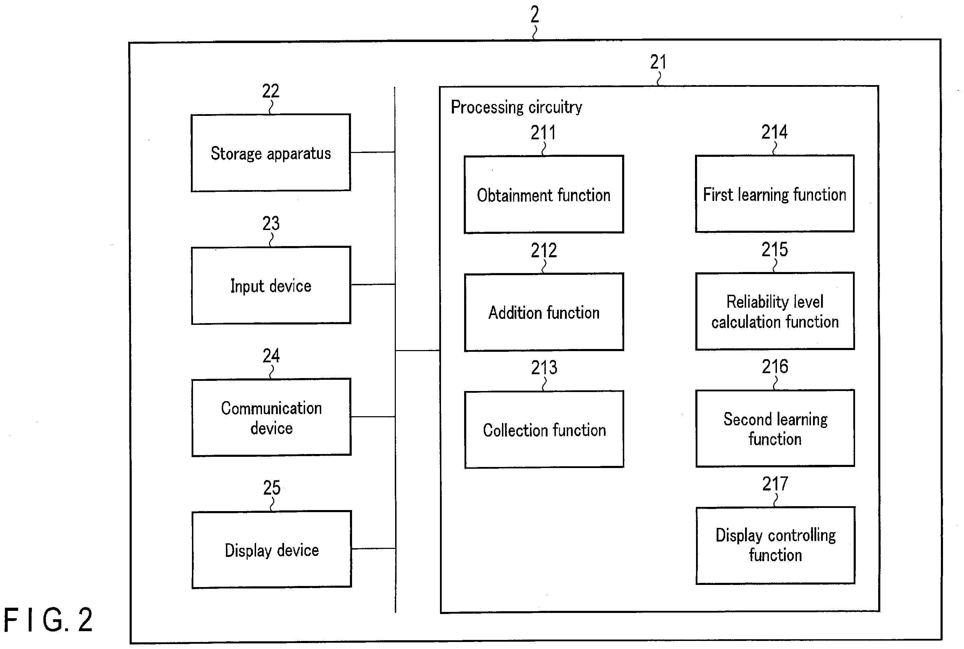

FIG. 2 is a diagram showing a configuration example of the medical information processing apparatus 2. As shown in FIG. 2, the medical information processing apparatus 2 has processing circuitry 21, a storage apparatus 22, an input device 23, a communication device 24, and a display device 25. The processing circuitry 21, the storage apparatus 22, the input device 23, the communication device 24, and the display device 25 are connected via a bus in such a manner that signals can be input to and output from each other.

The medical information processing apparatus 2 generates a machine learning model used for medical decision making (hereinafter, a "decision making model") and a machine learning model for determining a reliability level of a correct answer label (hereinafter a "reliability level determination model").

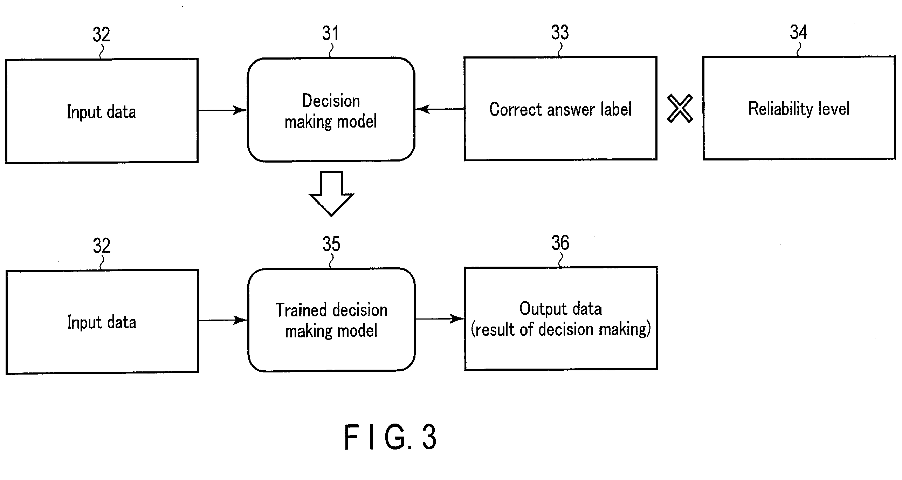

FIG. 3 is a diagram showing a relationship between the input and the output of the decision making model 31 in a learning phase and an operation phase. As shown in FIG. 3, in a learning phase, the decision making model 31 is trained based on the input data 32 of medical decision making, the correct answer label 33, and the reliability level 34 of the correct answer label 33. As the correct answer label 33, a correct answer label added by an operator's instruction that is input via the medical device terminal 3 is used. The reliability level 34 is an index value indicating a degree of reliability, such as appropriateness of the correct answer label 33. The reliability level 34 is used as a weight for the correct answer label 33. The reliability level 34 is output from the reliability level determination model. A combination of the input data 32, the correct answer label 33, and the reliability level 34 is called a "training sample". A plurality of training samples are collected for various kinds of input data. By training the decision making model 31 based on the plurality of training samples, a trained decision making model 35 is generated. In an operation phase, the input data 32 of medical decision making is input to the decision making model 35, and the decision making model 35 outputs output data 36 as a result of decision making.

The decision making model 31 and the trained decision making model 35 are a network having an input layer for inputting input data 32, hidden layers for converting input data 32 into output data 36, and an output layer for outputting output data 36. It suffices that there is at least one hidden layer. The decision making model 31 and the trained decision making model 35 are a multi-class classification model that outputs a probability of a corresponding class of multiple classes relating to a decision making result as the output data 36. For example, in the case of medical decision making for image diagnosis, a disease is placed as a class; in the case of medical decision making for determining an examination protocol, on the other hand, an examination protocol is placed as a class.

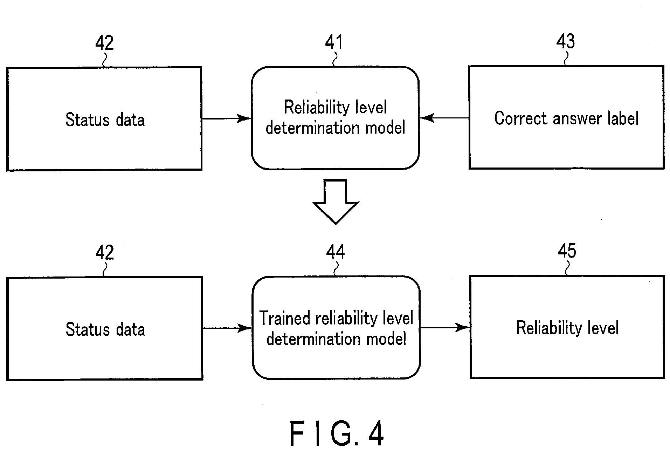

FIG. 4 is a diagram showing a relationship between the input and the output of the reliability level determination model 41 in a learning phase and an operation phase. As shown in FIG. 4, the reliability level determination model 41 is trained based on the status data 42 and the correct answer label 43 in a learning phase. The status data 42 is data indicating an operator's status while doing the work of adding a correct answer label 43. The status data 42 is data relating to an operator's operations, lines of sight, speech, and/or facial expressions that reflect a process of the operator's decision making at the time of adding a label. The correct answer label 43 is the same as the correct answer label 33 used in the decision making model 31, and the one added in accordance with an operator's instruction that is input via the medical device terminal 3 is used. A combination of the status data 42 and the correct answer label 43 is called a "training sample". A plurality of training samples are collected for various kinds of input data. By training the reliability level determination model 41 based on the plurality of training samples, a reliability level determination model 44 is generated. In an operation phase, the status data 42 is input to the reliability level determination model 44 and a reliability level 45 of the correct answer label is output from the reliability level determination model 44.

The reliability level determination model 41 and the trained reliability level determination model 44 are a neural network having an input layer for inputting the status data 42, hidden layers for converting the status data 42 into the reliability level 45, and an output layer for outputting the reliability level 45. It suffices that there is at least one hidden layer. The reliability level determination model 41 and the trained reliability level determination model 44 are a multi-class classification model that outputs a probability of a corresponding class of multiple classes relating to a decision making result as the reliability level 45. Specific tasks of the reliability level determination model 41 and the trained reliability level determination model 44 are set in accordance with those of the decision making model 31 and the trained decision making model 35. For example, if the tasks of the decision making model 31 and the trained decision making model 35 are medical decision making for image diagnosis, the tasks of the reliability level determination model 41 and the trained reliability level determination model 44 are also medical decision making for the image diagnosis; on the other hand, if the tasks of the decision making model 31 and the trained decision making model 35 are medical decision making for determining an examination protocol, the tasks of the reliability level determination model 41 and the trained reliability level determination model 44 are also medical decision making for determining an examination protocol.

As shown in FIG. 2, the medical information processing apparatus 2 has processing circuitry 21, a storage apparatus 22, an input device 23, a communication device 24, and a display device 25. The processing circuitry 21, the storage apparatus 22, the input device 23, the communication device 24, and the display device 25 are connected to each other via a bus in such a manner that signals can be mutually input and output.

The processing circuitry 21 includes processors such as a CPU (central processing unit) and a GPU (graphics processing unit). The processing circuitry 21 executes a medical information processing program to realize an obtainment function 211, an addition function 212, a collection function 213, a first learning function 214, a reliability level calculation function 215, a second learning function 216, and a display controlling function 217, etc. Note that the embodiment is not limited to the case in which the respective functions 211 to 217 are realized by single processing circuitry. Processing circuitry may be composed by combining a plurality of independent processors, and the respective processors may execute programs, thereby realizing the functions 211 to 217. The functions 211 to 217 may be respective modularized program constituting a consensus making support program or separate programs. These programs are stored in the storage apparatus 22.

By the realization of the obtainment function 211, the processing circuitry 21 obtains various information items. For example, the processing circuitry 21 obtains input data of medical decision making. The input data of medical decision making is used for training a decision making model. The input data of medical decision making can be obtained from a medical information system, such as a hospital information system (HIS) or a radiology information system (RIS), etc.

Through realization of the addition function 212, the processing circuitry 21 adds a correct answer label in accordance with an operator's instruction that is input via the medical device terminal 3. The correct answer label is used for training a reliability level determination model and a decision making model.

By the realization of the collection function 213, the processing circuitry 21 acquires various information items. For example, the processing circuitry 21 collects status data indicating an operator's status while doing the work of adding a correct answer label.

Through realization of the first learning function 214, the processing circuitry 21 trains, based on the status data and the correct answer label, a reliability level determination model which accepts the status data and outputs a reliability level of the correct answer label.

Through realization of the reliability level calculation function 215, the processing circuitry 21 calculates a reliability level of a correct answer label based on the trained reliability level determination model.

Through realization of the second learning function 216, the processing circuitry 21 trains the decision making model which accepts the input data and outputs output data that is data indicating a result of the decision making, based on the input data of medical decision making, the correct answer label, and the reliability level.

Through realization of the display controlling function 217, the processing circuitry 21 display various information on the display device 25 and/or the medical device terminal 3. For example, the processing circuitry 21 display a reliability level obtained by the reliability level calculation function 215, etc, on the display device 25 and/or the medical device terminal 3.

The storage apparatus 22 is a ROM (read only memory), a RAM (random access memory), an HDD (Hard Disk Drive), an SSD (Solid State Drive), or an integrated circuit storage device, etc. storing various types of information. The storage apparatus 22 may be not only the above-listed memory devices, but also a driver that writes and reads various types of information to and from, for example, a portable storage medium such as a compact disc (CD), a digital versatile disc (DVD), a flash memory, or a semiconductor memory. The storage apparatus 22 may be provided in another computer connected via a network.

The input device 23 accepts various kinds of input operations from an operator, converts the accepted input operations to electric signals, and outputs the electric signals to the processing circuitry 21. Specifically, the input device 23 is connected to an input device, such as a mouse, a keyboard, a trackball, a switch, a button, a joystick, a touch pad, or a touch panel display. The input device 23 outputs to the processing circuitry 21 an electrical signal corresponding to an input operation on the input device. The input device 23 may be an input device provided in another computer connected via a network or the like.

The communication device 24 is an interface for sending and receiving various types of information to and from other computers, such as the medical device terminal 3, etc. included in the medical information processing system 1.

The display device 25 displays various types of information through the display controlling function 217 of the processing circuitry 21. For the display device 25, for example, a liquid crystal display (LCD), a cathode ray tube (CRT) display, an organic electro luminescence display (OELD), a plasma display, or any other display can be used as appropriate. A projector may be used as the display device 25.

Next, an operation example of the medical information processing apparatus 2 is described. In the description hereinafter, assume that a task of the decision making model is determining an examination protocol. A decision making model that determines an examination protocol will be hereinafter called an "examination protocol classification model".

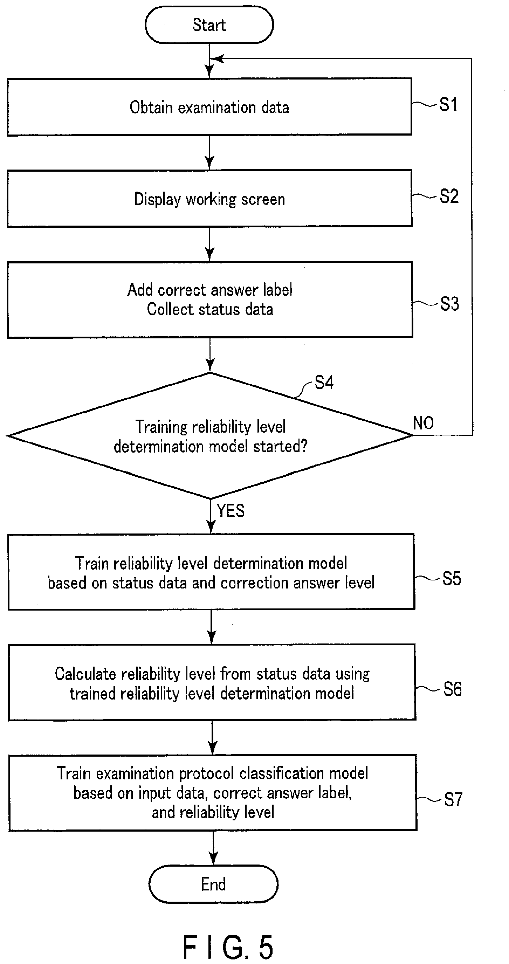

FIG. 5 is a diagram showing a flow of an example of medical information processing by the medical information processing apparatus 2. As shown in FIG. 5, the processing circuitry 21 obtains examination data through the realization of the obtainment function 211 (step S1). In step S1, the processing circuitry 21 obtains examination data relating to a patient targeted for medical decision making. In the present embodiment the medical decision making is an examination protocol determination; therefore, examination data is obtained as data that may be referred to when an examination protocol is determined. The examination data is obtained from a hospital information system, such as a hospital information system (HIS) or a radiology information system (RIS), etc. A part or an entirety of the examination data is used as input data in medical decision making.

After step S1, the processing circuitry 21, through the realization of the display controlling function 217, displays a working screen for examination protocol determination (step S2). In step S2, the processing circuitry 21 displays the working screen with a predetermined layout.

After step S2, the processing circuitry 21 adds a correct answer label through the implementation of the addition function 212 (step S3). In step S3, the processing circuitry 21 adds a correct answer label on the working screen displayed in step S2, in accordance with an operator's instruction that is input via the input device 23 and/or the medical device terminal 3. As a correct answer label, an examination protocol is added. At the time of adding a correct answer label, the processing circuitry 21 collects status data indicating a status of the operator while doing the work of the adding a correct answer label, through the implementation of the collection function 213. The processing circuitry 21 collects, as status data, data relating to operator's operations, lines of sight, speech, and/or facial expressions that reflect a process of the operator's decision making at the time of adding a correct answer label.

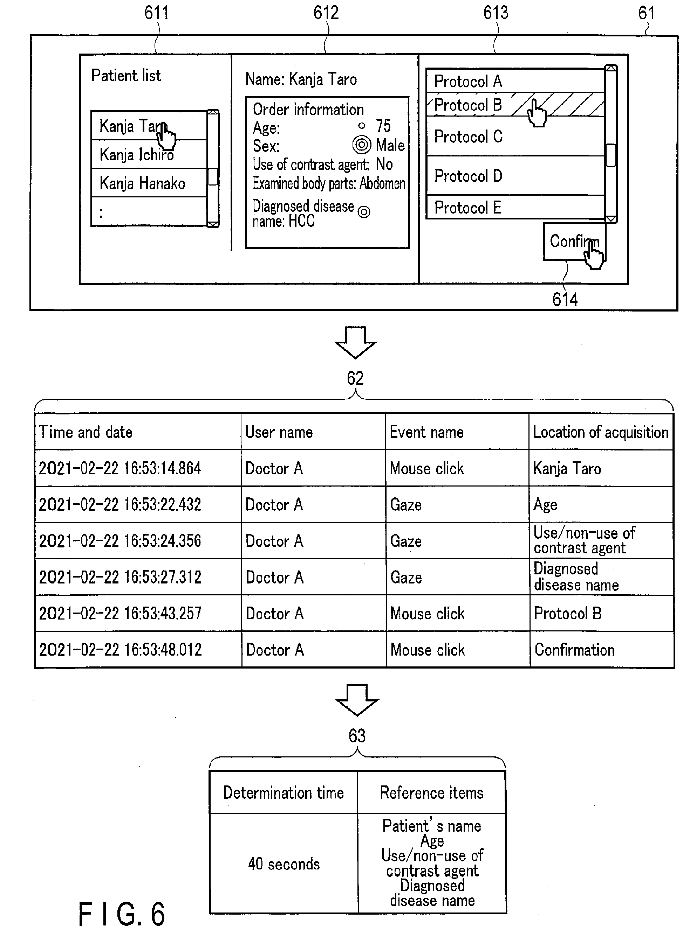

FIG. 6 is a diagram schematically showing adding of a correct answer label and collection of status data in step S3. As shown in FIG. 6, a working screen 61 for adding a correct answer label is displayed on the display device 25. The working screen 61 includes a select area 611 for selecting a target patient who is a target for determining an examination protocol, a display area 612 of examination data relating to the target patient, and a select area 613 for selecting an examination protocol which is a correct answer label. A list of names of candidate patients is displayed in the select area 611 in such a manner that each patient name is selectable. FIG. 6 shows an example where "Kanja Taro" is selected as a target patient. Examination data relating to the target patient "Kanja Taro" is displayed in the display area 612. Specifically, age, sex, use or non-use of a contrast agent, an examined body parts, a diagnosed disease, etc. are displayed as order information in the examination data. The select area 613 displays a list of examination protocol names or symbols in such a manner that each examination protocol is selectable. FIG. 6 shows that "protocol B" is selected as a correct answer label. After an examination protocol is selected, the confirmation button is pressed via the input device 23.

At the time of an operator adding a correct answer label, the processing circuitry 21 collects an event log 62 that can be obtained through the working screen 61 that occurs during a process of determining a correct answer label by the operator. The event log 62 is raw data relating to the status data. As an example, an operation log of a screen operation by an operator via the input device 23 and a log of an operator's line of sight on the working screen 61 are collected as event logs 62.

As shown in FIG. 6, the event logs 62 are recorded in a chronological manner for items such as date and time, user name, event name, and location of acquisition. Date and time is a time when the event took place. A user name is a name of the operator who added the correct answer label. Event name indicates a type of event. A location of acquisition indicates a location where the event takes place on the working screen. As an example, for the case where the user "Doctor Taro" selected "Kanja Taro" on the select area 611 using a mouse at a date and time of "2021-02-22, 16:53:14.854", the event name "mouse clicking" and the location of acquisition "Kanja Taro" are recorded as operation logs. As another example, for the case where the user "Doctor Taro" was looking at "age" displayed on the display area 612 at the date and time "2021-02-22, 16:53:22.432", the event name "looking" and the location of acquisition "age" are recorded as line-of-sight logs. Whether the operator looks or not may be determined based on whether or not the operator's line of sight stays on the same displayed item on the working screen 61 longer than a predetermined length of time. The display item located at the arrival point of the operator's line of sight can be calculated based on a correspondence between the location of the operator's eyeballs reflected on an image taken by an optical camera arranged in the display device 25, etc. and the location of each item on the working screen 61.

The event logs 62 are recorded for each targeted patient. Specifically, the events of selecting a targeted patient to adding a correct answer label, in other words pressing the confirmation button, are collected as a single event log 62 relating to the target patient. The speech log, which is a log relating to an operator's speech, and/or a facial expression log, which is a log relating to an operator's facial expression, may be collected as an event log 62. It suffices that a speech log is collected by performing speech recognition on audio signals collected by a microphone and converting the signals into text information. It suffices that a facial expression log is collected by analyzing an operator's facial expression reflected on an image taken by an optical camera.

After event logs 62 relating to the target patient are collected, the processing circuitry 21 generates status data 63 based on the event logs 62. The status data 63 has determination time information and reference items as an example. The determination time information is time relating to a time required for the work of adding a correct answer label. More specifically, it is an elapsed time from the time when the target patient is selected to the time when the confirmation button of an examination protocol is pressed. The reference item is information relating to a display item that the operator looks at in the examination data. More specifically, information of a location of acquisition relating to the event name "looking". The status data 63 relating to the target patient is stored in the storage apparatus 22. A combination of the status data 63 relating to the target patient and the correct solution label is stored as a single training sample in the storage apparatus 22.

The determination time may be fragmented according to reference items when it is recorded. For example, x seconds from a selection of a patient to checking of patient information, y seconds from checking of patient information to confirmation of a protocol. The attention level may be converted into a numerical value. For example, three seconds for determining whether to use a contrast agent, ten seconds to refer to a diagnosed disease name, three times of checking (rechecking) a use of a contrast agent in a single diagnosis session.

After step S3, the processing circuitry 21 determines whether or not the training of the reliability level determination model should be started through realization of the first learning function 214 (step S4). In step S4, the processing circuitry 21 determines whether or not the number of collected training samples has reached the number required to train the reliability level determination model. If the number of collected training samples is less than the required number, the training of the reliability level determination model is not started, and steps S1 through S3 are repeated for another patient.

Then, if the number of collected training samples has reached the required number, and it is determined that training of the reliability level determination model is to be started (Yes in step S4), the processing circuitry 21 trains the reliability level determination model based on status data and the correct answer label through a realization of the first learning function 214 (step S5).

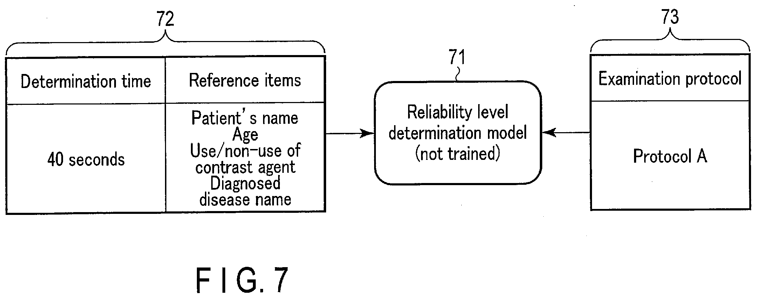

FIG. 7 is a diagram showing a relationship between the input and the output of the reliability level determination model 71 in a learning phase. As shown in FIG. 7, the processing circuitry 21 trains the learning parameter of the reliability level determination model 71, which is a multi-class classification model, based on the supervised learning in which the status data 72 is input and the correct answer label 73 is used as a teacher. It is desirable that classes be provided in accordance with examination protocol candidates. For example, if there are three candidates, "protocol A", "protocol B", and "protocol C", three classes are provided. In FIG. 7, the status data 72 includes a determination time ("40 seconds") and reference items ("patient's name", "age", "use/non-use of contrast agent", "diagnosed disease name"), and the correct answer label 73 includes an examination protocol ("protocol A"). In the training process, the processing circuitry 21 calculates a prediction label obtained by inputting the status data 72 to the reliability level determination model 71 and performing forward propagation computation, and an error between the prediction label and the correct answer label 73 is calculated, and updates a learning parameter by optimizing the error using a method such as stochastic gradient descent. A prediction label is an output of the reliability level determination model 71 and is a vector amount indicating the probability of each class. The learning parameter are optimized by repeating the updating calculation using a plurality of training samples in such a manner that the error is minimized. A trained reliability level determination model is generated by allocating the optimized learning parameter to the machine learning model. The learning parameter is a weight variable or a bias, etc. indicating conversion between layers included in the reliability level determination model 71.

As described above, by training the reliability level determination model 71 based on supervised learning in which the status data 72 is input and the correct answer label 73 is used as a teacher, the reliability level determination model 71 learns a correlation between the status data 72 and the correct answer label 73. Herein, the correlation between the status data 72 and the correct answer label 73 is explained.

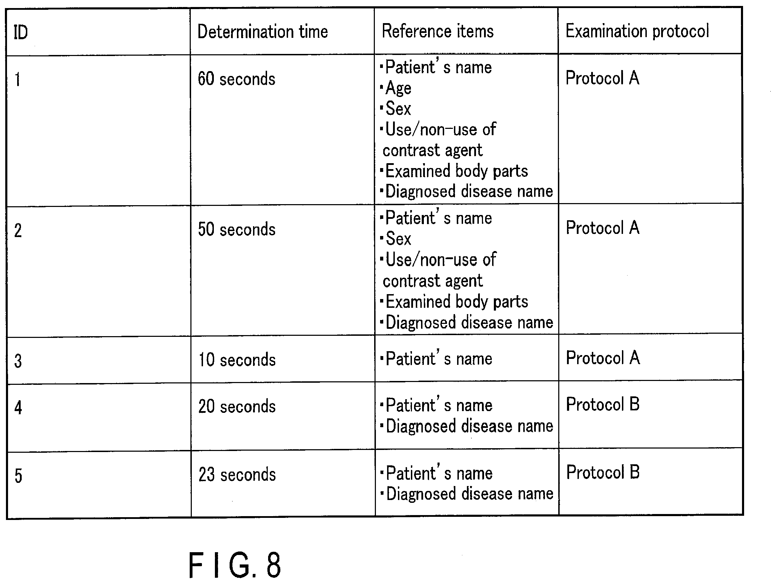

FIG. 8 is a diagram showing an example of training data. As shown in FIG. 8, the training data includes status data (determination time and reference items) and the correct answer label (examination protocol). It is understood from the training data that an operator tends to take a relatively a long time and refer to more information items, such as a use or non-use of a contrast agent and an examined body parts, to choose protocol A. On the other hand, an operator tends to take a shorter time and refer to fewer information items to choose protocol B. The reliability level determination model learns these tendencies. For example, from the training data, it is understood that an operator chooses protocol B in a relatively short time of 20 seconds after checking the patient's name and the diagnosed disease name only. If a short determination time and fewer reference items are included in the status data, the reliability level determination model outputs a higher value of the probability of protocol B than the probability of protocol A. The probability of each protocol indicates a degree of appropriateness of choosing the protocol in consideration of the input status data, in other words, the degree of reliability of the protocol. In the present embodiment, the probability is used as a reliability level for labelling.

After step S5, the processing circuitry 21 calculates a reliability level from the status data, using the trained reliability level determination mode, through realization of the reliability level calculation function 215 (step S6).

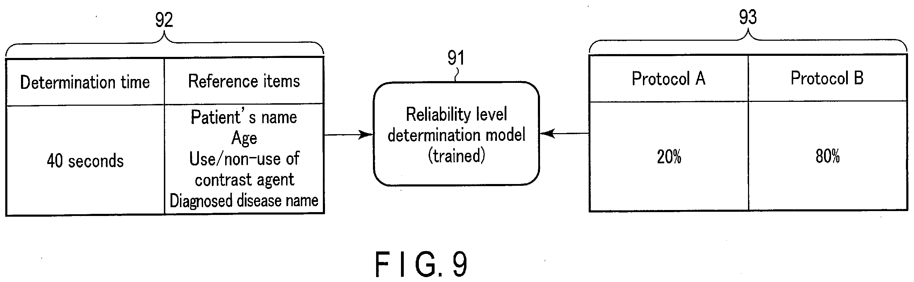

FIG. 9 is a diagram showing a relationship between the input and the output of the trained reliability level determination model 91 in an operation phase. As shown in FIG. 9, the status data 92 is input to the reliability level determination model 91 and the model outputs the reliability level 93. As described above, suppose that the reliability level determination model 91 is a class classification model, and there are two classes, protocol A and protocol B. The reliability level determination model 91 outputs a probability (likelihood) of each protocol. The probability is used as a reliability level. In FIG. 9, similarly to the status data 72 in FIG. 7, the status data 92 includes the determination time "40 seconds", the reference items "patient's name", "age", "use/non-use of contrast agent", "diagnosed disease name", and the reliability level 93 includes protocol A "20%" and protocol B "80%".

In step S6, the processing circuitry 21 calculates a reliability level for status data of each target patient. The reliability level is registered in the reliability level database being associated with the status data.

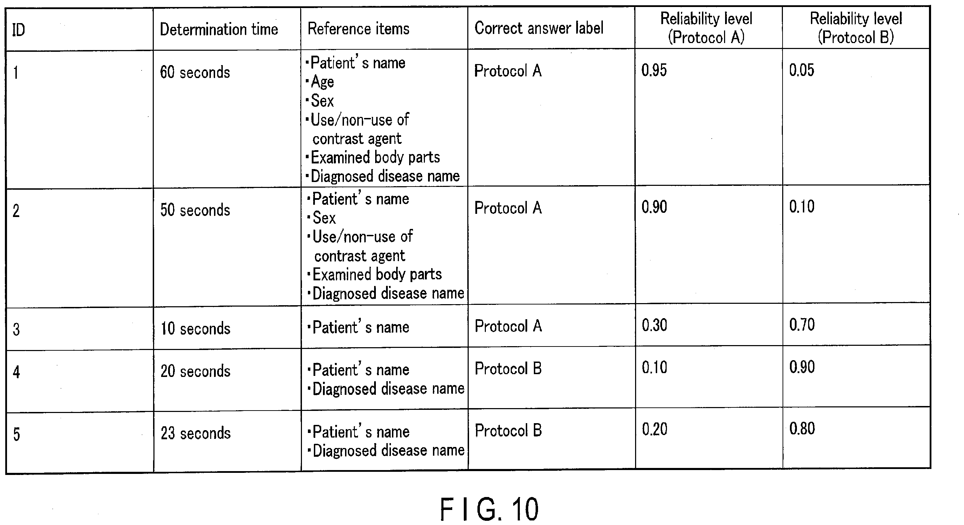

FIG. 10 is a diagram showing an example of a reliability database. As shown in FIG. 10, the status data, the correct answer label, and the reliability level are associated with each other in the reliability level database. The reliability level is registered for each of protocol A and protocol B. For example, for ID "1", protocol A "0.95" and protocol B "0.05" are registered as a reliability level.

After step S6, the processing circuitry 21 trains the examination protocol classification model based on the input data and the correct answer label through realization of the second learning function 216 (step S7). The input data is data input to the examination protocol classification model and is a part of the examination data. In other words, the input data is data of items for which a correlation can be acknowledged between the item and the examination protocol in the examination data. The items of the input data are predetermined. Assume that the input data is associated with the status data, the correct answer label, and the reliability level through an ID, etc.

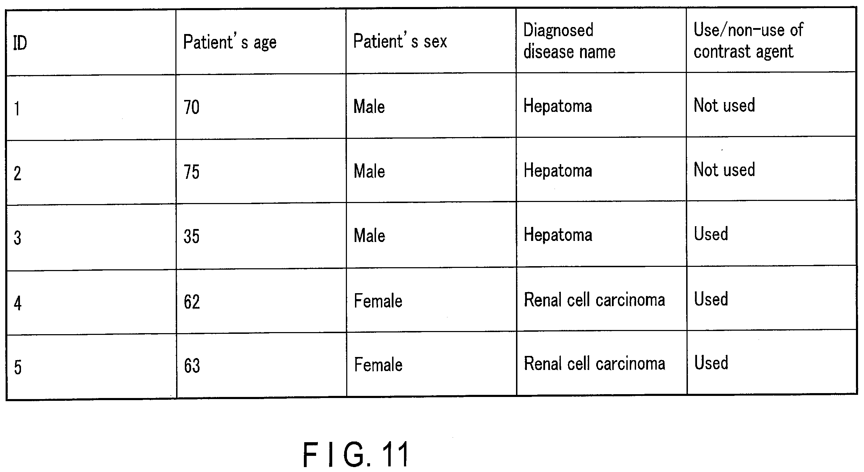

FIG. 11 is a diagram showing an example of input data. As shown in FIG. 11, the input data relates to patient's age ("70", "75", etc.), patient's sex ("male", "female", etc.), diagnosed disease name ("hepatoma", "renal cell carcinoma", etc.) and use/non-use of a contrast agent ("used" or "not used").

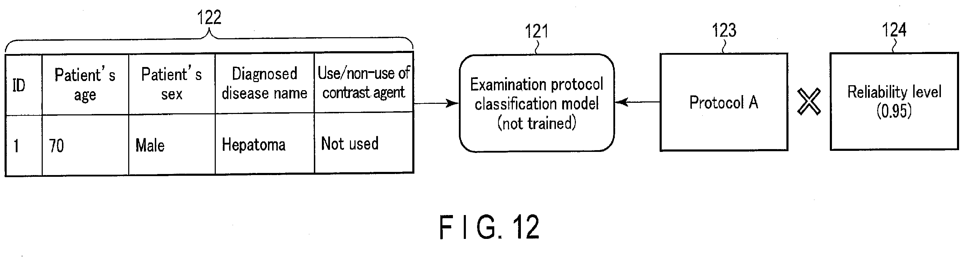

FIG. 12 is a diagram showing a relationship between the input and the output of the examination protocol classification model 121 in a learning phase. The processing circuitry 21 specifies the input data 122 targeted for processing, and extracts the correct answer label 123 and the reliability level 124 associated with the targeted input data 122 from the reliability level database. As shown in FIG. 12, the processing circuitry 21 trains the learning parameter of the examination protocol classification model 121, which is a multi-class classification model, based on supervised learning in which the input data 122 is input and the correct answer label 123 weighted by the reliability level 124 is used as a teacher. In FIG. 12, the input data 122 is: patient's age "70", patient's sex "male", diagnosed disease name "hepatoma", and use/non-use of a contrast agent "not used", and the correct answer label 73 is "protocol A", and the reliability level 124 is "0.95".

In the training process, the processing circuitry 21 calculates an output obtained by performing forward propagation computation (hereinafter, a "model output") on the input data 122 that is input to the examination protocol classification model 121, and the processing circuitry 21 calculates an error between the model output and the correct answer label 73, and updates the learning parameter by optimizing the error by, for example, stochastic gradient descent. The learning parameter is optimized by repeating the updating calculation using a plurality of training samples in such a manner that the error is minimized.

Herein, the error is expressed by a loss function L(T,p,w) wherein T is a correct answer label, p is a model output, and w is a reliability level of the correct answer label T, as represented by Expression (1) below. The subscript k indicates a training sample number.

$$L(T, p, w) = -\sum_k w_k T_k \log(p_k) \qquad (1)$$

As represented by Expression (1), the loss function L (T,p,w) is defined by a total sum of cross entropy indicating an error between a correct answer label T weighted by a reliability level w and a model output p over the plurality of training samples. As an example, in the case of the training samples shown in FIG. 12, since the reliability level 124 of the correct answer label 123 is "0.95", when the cross entropy is computed, "0.95" is multiplied by the value "1.00" of the correct answer label 123 "protocol A". The training parameter of the examination protocol determination model is trained so that the loss function L(T,p,w) is minimized over the plurality of training samples k. The trained examination protocol classification model is generated by allocating the determined parameter that is optimized so as to minimize the loss function L(T,p,w) to the machine learning model.

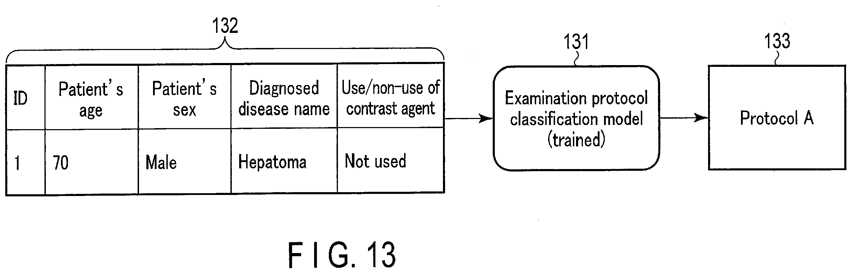

FIG. 13 is a diagram showing the relationship between the input and the output of the trained examination protocol classification model 131 in an operation phase. As shown in FIG. 13, the input data 132 is input to the examination protocol classification model 131 and the model outputs the examination protocol 133. Specifically, a probability of each of protocol A and protocol B and the name of the protocol with a larger probability is output as the examination protocol 133. The examination protocol classification model 131 may be designed to output a probability of each of protocol A and protocol B.

After step S7, the medical information processing according to the first embodiment is finished.

The above-described flow of the medical information processing is an example, and the present embodiment is not limited to this. In the foregoing description the processing circuitry 21 successively performs the training of the reliability level determination model (S5) and the training of the examination protocol classification model (S7); however, for example, the training of the examination protocol classification model (S7) may be performed after a predetermined length of time, for example a few days, a few weeks, or a few months, of the training of the reliability level determination model (S5). The reliability level determination model (S5) and the training of the examination protocol classification model (S7) may not be necessarily performed by the same medical information processing apparatus 2 and may be performed by separate medical information processing apparatuses. The plurality of training samples (correct answer label and status data) are not necessarily collected by the same medical information processing apparatus 2 and may be collected by separate medical information processing apparatuses.

According to the foregoing embodiment, the medical information processing apparatus 2 has the addition function 212, the collection function 213, the first learning function 214, the obtainment function 211, and the second learning function 216. The addition function 212 adds a correct answer label used for training a decision making model, which is a machine learning model used for decision making by health care provision, in accordance with an operator's input instruction. The collection function 213 collects status data indicating a status of the operator while doing the work of adding a correct answer label. The first learning function 214 trains a reliability level determination model, which is a machine learning model to which status data is input and which outputs a reliability level of the correct answer label, based on the status data and the correct answer label. The obtainment function 211 obtains input data of the decision making model. The second learning function 216 trains the decision making model to which the input data is input and which outputs output data that is data indicating a result of the decision making, based on the input data, the correct answer label, and the reliability level.

According to the above structure, it is possible to evaluate the reliability level of each correct answer level based on the status data indicating an operator's status while doing the work adding a correct answer label. Since the decision making model is trained based on a correct answer label evaluated with a reasonable reliability level, a correct answer label having a low reliability level does not contribute to the training; it is thus possible to increase prediction accuracy of the decision making model.

The foregoing medical information processing is merely an example, and the present embodiment is not limited to this example and can be modified in various ways.

(Modification 1)

In the above-described example, the reliability level determination model is trained based on status data and a correct answer label. However, the present embodiment is not limited thereto. Hereinafter, training of the reliability level determination model according to Modification 1 is described.

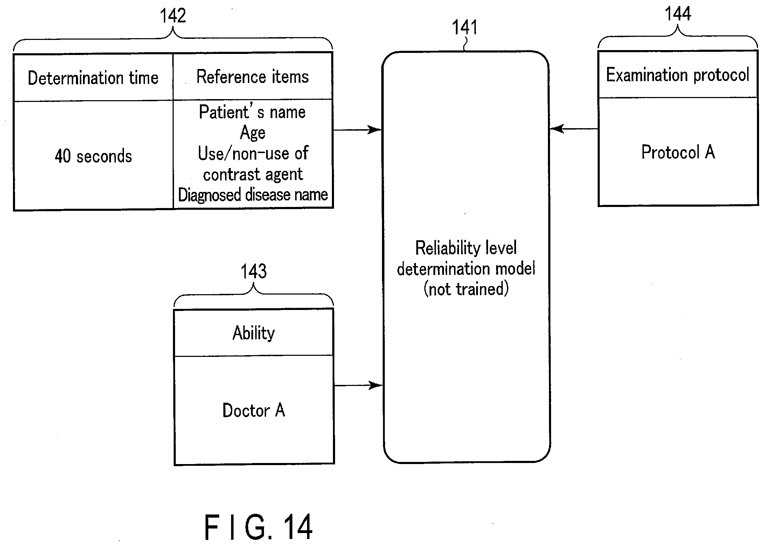

FIG. 14 is a diagram showing the relationship between the input and the output of the reliability level determination model 141 according to Modification 1 in a learning phase. As shown in FIG. 14, the processing circuitry 21 trains a training parameter of the reliability level determination model 141, which is a multi-class classification model, to which status data 142 and the ability data 143 are input, based on supervised training in which the correct answer label 144 is used as a teacher. The status data 142 is data indicating an operator's status while doing the work of adding a correct answer label, similarly to the foregoing example. The correct answer label 144 is a label added by the operator, similarly to the foregoing example. The ability data 143 is data indicating an ability of the operator. Specifically, a name of the operator is used as the ability data 143, as shown in FIG. 14. The ability data 143 is not limited to an operator's name, and some kind of data having a correlation to an ability, such as the number of years of being employed, the number of years of experience, a job title, and the like, may be used.

During the training process, the processing circuitry 21 calculates a predicted label obtained by inputting the status data 142 and the ability data 143 to the reliability level determination model 141 and performing forward propagation computation, calculates an error between the prediction label and the correct answer label 144, and updates a training parameter in accordance with an optimization method, such as stochastic gradient descent. The learning parameter is optimized by repeating the updating calculation using a plurality of training samples in such a manner that the error is minimized. A trained reliability level determination model is generated by allocating the optimized learning parameter to the machine learning model. With the above training method, a reliability level determination model to which status data and ability data are input and which outputs a reliability level can be generated. The reliability level is used as a weight in the training of the examination protocol classification model, similarly to the above-described example.

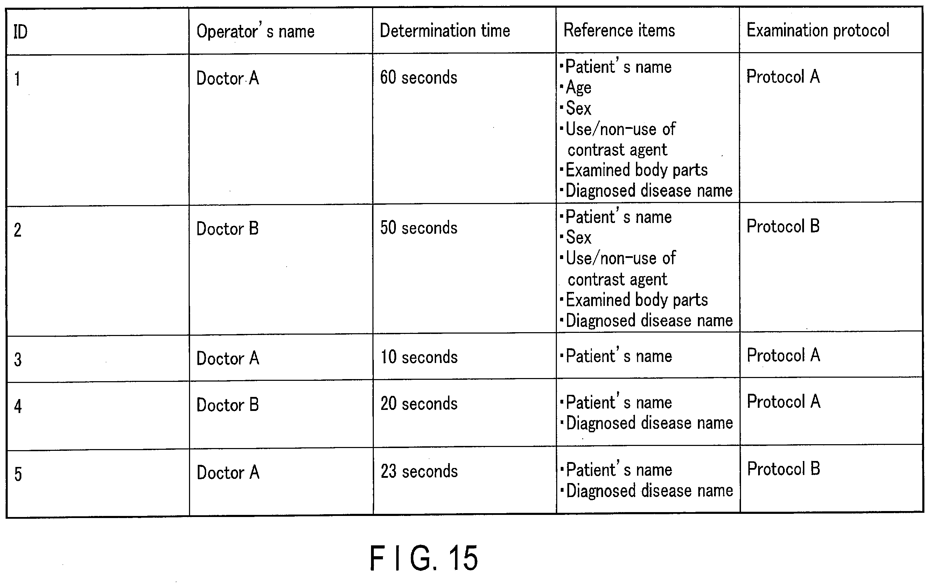

FIG. 15 is a diagram showing an example of training data according to Modification 1. As shown in FIG. 15, the training data includes ability data (operator's name), status data (determination time and reference items), and the correct answer label (examination protocol). From the training data, doctor A tends to select protocol A when he/she refers to more reference items and takes a longer determination time, whereas doctor B tends to select protocol B when he/she refers to more reference items and takes a longer determination time. Thus, the ability data also has a correlation between a correct answer label and a reliability level, and if the reliability level determination model is trained using the ability data in addition to the status data, it is thus possible to improve the accuracy of the reliability level.

(Modification 2)

Hereinafter, training of the reliability level determination model according to Modification 2 is described.

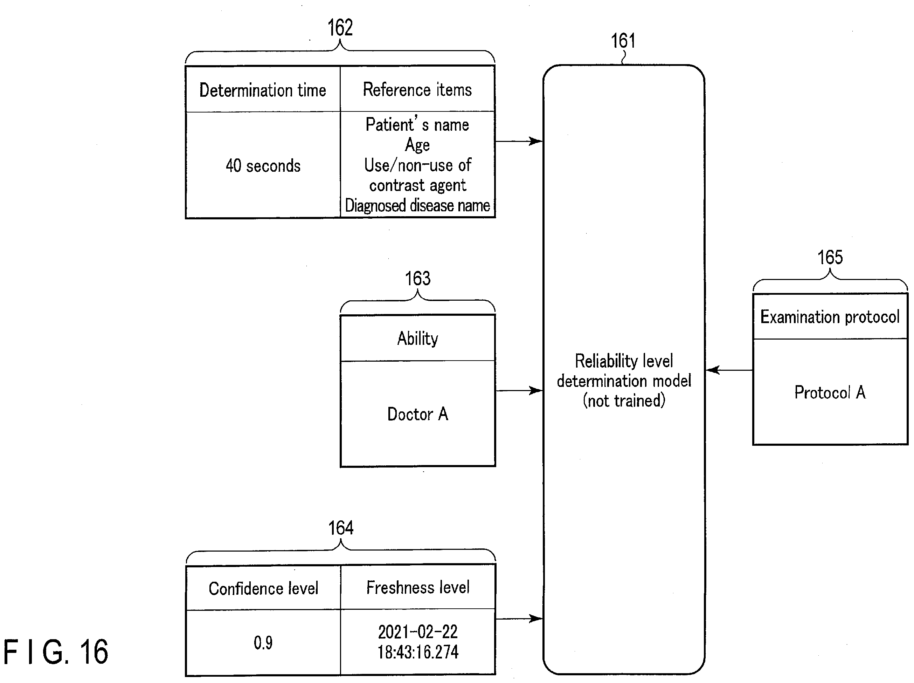

FIG. 16 is a diagram showing the relationship between the input and the output of the reliability level determination model 161 according to Modification 2 in a learning phase. As shown in FIG. 16, the processing circuitry 21 trains a training parameter of the reliability level determination model 161, which is a multi-class classification model, to which status data 162, the ability data 163, and the additional data 164 are input, based on supervised training in which the correct answer label 165 is used as a teacher. The status data 162 is data indicating an operator's status while doing the work of adding a correct answer label, similarly to the foregoing example. The ability data 163 is data indicating an ability of the operator, similarly to Modification 1. The correct answer label 165 is a label added by the operator, similarly to the foregoing example. The additional data 164 is data obtained before and after the work of adding a correct answer label and is expected to have a correlation to the reliability level. Specifically, the additional data 164 is data related to a freshness level, a confidence level, quality, and/or a required time. FIG. 16 shows a freshness level and a confidence level as the additional data 164.
A freshness level is a time at which the operator adds a correct answer label. The later the time is, the higher the freshness level is. The higher the freshness level is, the higher the reliability level is. The confidence level indicates a degree of subjective confidence felt by an operator toward a correct answer label added by himself/herself or when he/she adds a label. The confidence level is input by an operator after a correct answer label is added. For example, if the operator is aware that he/she took some time to add a correct answer label, a lower confidence level is assigned compared to that assigned to an operator who is aware that he/she did not take very much time. The higher the confidence level is, the higher the reliability level is. The quality indicates the quality of information which is referred to when a correct answer label is added. For example, if an annotation is added to a medical image as a correct answer label, it is information regarding a quality of the medical image. As quality, an image format of a medical image, a presence/absence of artifacts in a medical image, a difference in SNR, and an imaging condition are used. A required time is a difference between an optimal value of a preset required time (determination time) and an actual required time (determination time) or a score based on the difference.
During the training process, the processing circuitry 21 calculates a predicted label obtained by inputting the status data 162, the ability data 163, and the additional data 164 to the reliability level determination model 161 and performing forward propagation computation, calculates an error between the prediction label and the correct answer label 165, and updates a training parameter in accordance with an optimization method, such as stochastic gradient descent. The learning parameter is optimized by repeating the updating calculation using a plurality of training samples in such a manner that the error is minimized. A trained reliability level determination model is generated by allocating the optimized learning parameter to the machine learning model. With the above training method, a reliability level determination model to which the status data 162, the ability data 163, and the additional data 164 are input and which outputs a reliability level can be generated. The reliability level is used as a weight in the training of the examination protocol classification model, similarly to the above-described example.
By training a reliability level determination model using additional data in addition to the status data and the ability data, it is expected that the accuracy of the reliability level is improved compared to Modification 2.

(Modification 3)

In the foregoing various examples, the reliability level that is output from the reliability level determination model is used as a weight in the training of the decision making model, such as an examination protocol classification model, etc. However, the present embodiment is not limited thereto. The processing circuitry 21 according to Modification 3 displays a reliability level. Hereinafter, the display of a reliability level is explained.
Suppose that the decision making model according to Modification 3 is an image diagnosis model to which a medical image is input and which outputs an annotation indicating a disease candidate area. An annotation is included in a correct answer label. More specifically, the imaging model is a multi-class classification model that outputs a probability of a disease candidate for each unit area such as a pixel. The image diagnosis model outputs the probability of each disease candidate in each unit area, and outputs a disease candidate with the highest probability. The processing circuitry 21 retains a color table defining a correspondence between a disease candidate and a color value, determines a disease candidate with a highest probability for each unit area, and displays the unit area with a color value corresponding to the disease candidate. A set of unit areas each displayed with a color value corresponding to a disease candidate constitutes an annotation.
An annotation is used as a correct answer label in order to train an image diagnosis model; however, similarly to the forgoing example, the operator adds the annotation. The reliability level determination model according to Modification 3 is trained based on status data relating to the time when an annotation is added and an annotation, and state data is input to the model and the model outputs a reliability level of each unit area. The processing circuitry 21 causes the display device 25 to display a medical image on which a reliability level is overlaid.

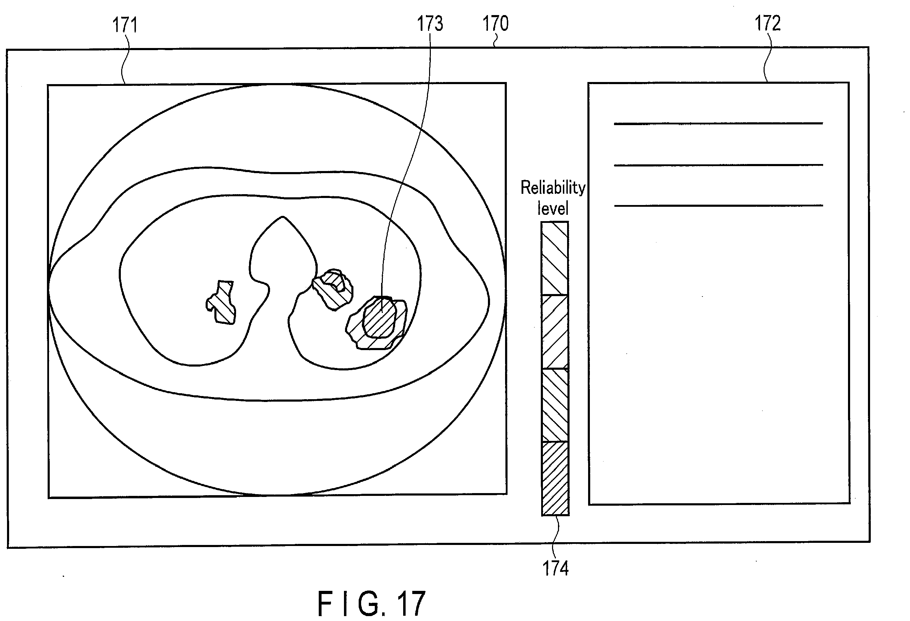

FIG. 17 is a diagram showing an example of a display screen 170 for the reliability level. As shown in FIG. 17, the display screen 170 displays a medical image 171 and an information display section 172. The medical image 171 is a medical image which is a target for adding an annotation, which is a correct answer label. The annotation 173 added by the operator is overlaid on the medical image 171. A color value (color code) according to a reliability level determined for each pixel by the reliability level determination model is assigned to each pixel of the annotation 173, and the annotation 173 is displayed in a color value according to the reliability level. The correspondence between the reliability level and the color value is defined by the color table 174, and it is preferable to display the correspondence on the display screen 170 so that the operator, etc. can know the relationship between the reliability level and the color value. In the text information display section 172, examination data, etc. of a patient who is a subject of the medical image 171, which was referred to when an annotation 173 was added, is displayed.

Typically, the display screen 170 is observed by the operator who added the annotation 173. By displaying the annotation 173 with color coding in accordance with a reliability level, it is possible to easily check the reliability level for each area of the annotation 173. For example, it is possible to prompt an operator to review the annotation for the area where the reliability level is low.

The correspondence between the reliability level and the color value may be designed freely at request. In the example shown in FIG. 17, the reliability level is divided by a discretionarily selected number of color values, for example 4. In this case, it suffices that the range of reliability level for each color value may be set by equally dividing the range of values that may be taken by the reliability level (for example, from "0" to "1").

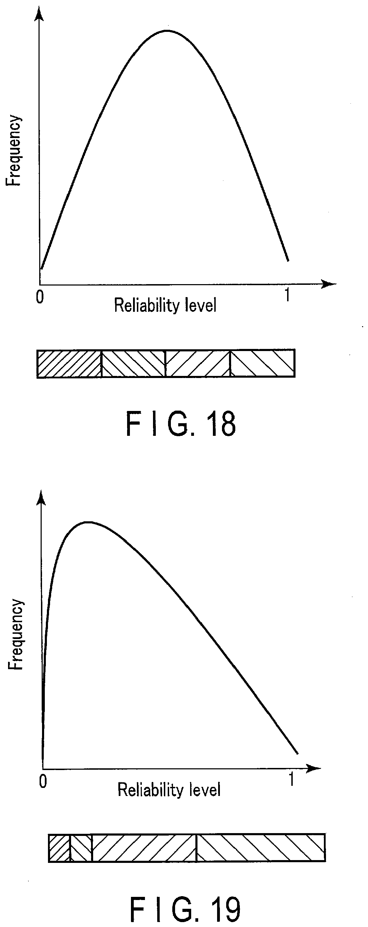

FIG. 18 is a diagram showing an example of a correspondence between frequency distribution of a reliability level and a color value. FIG. 19 is a diagram showing another example of a correspondence between frequency distribution of a reliability level and a color value. As shown in FIGS. 18 and 19, the correspondence between the reliability level and the color value may be set in accordance with the frequency distribution of the reliability level. As an example, a color value is set based on the reliability level and the frequency. If there are four categories of the color value, the categories can be divided into four groups, namely a group ranging from the lowest to the middle frequencies, a group ranging from the middle to the highest frequencies, a group ranging from the highest to the middle frequencies, and a group ranging from the middle to the lowest frequencies. As shown in FIG. 18, in the case where the frequency of reliability level is equally distributed around the intermediate value of "0.5", the category of the color value is set within the range obtained by equally dividing the range that may be taken by the reliability level (for example, from "0" to "1").

As shown in FIG. 19, if the difficulty level of adding an annotation is relatively high, the reliability level is heavily distributed on the side of a low reliability level, and the category of the color value is minutely set for a lower reliability level. In other words, the processing circuitry 21 sets the correspondence between the reliability level and the color value in accordance with a level of difficulty in adding an annotation. In the case where the number of color value categories is finite, it is possible to set minute color value categories of color value for the range of reliability level to the range of reliability level that needs to be focused on in accordance with the difficulty level.

The display of the reliability level is not limited to a case where the decision making model is an image diagnosis model; any type of model, such as an examination protocol classification model can be used.

According to at least one of the above-explained embodiments, it is possible to improve an accuracy in prediction by a machine learning model.

The term "processor" used in the above explanation indicates, for example, a circuit, such as a CPU, a GPU, or an Application Specific Integrated Circuit (ASIC), and a programmable logic device (for example, a simple programmable logic device (SPLD), a complex programmable logic device (CPLD), and a field programmable gate array (FPGA)). The processor realizes its function by reading and executing the program stored in the storage circuitry. The program may be directly incorporated into the circuit of the processor instead of being stored in the storage circuit. In this case, the processor implements the function by reading and executing the program incorporated into the circuit. The function corresponding to the program may be implemented by a combination of logic circuits instead of executing the program. The processors described in connection with the above embodiments are not limited to single-circuit processors; a plurality of independent processors may be integrated into a single processor that implements the functions of the processors. Furthermore, a plurality of constituent elements shown in FIGS. 1 and 2 may be integrated into one processor to implement the functions.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

**Claims**

1. A medical information processing apparatus comprising:

   an addition unit (212) configured to add, in accordance with an operator's input instruction, a correct answer label used for training a machine learning model to which medical data is input and which outputs a label indicating a character or area used for a medical diagnosis corresponding to the input medical data, the correct answer label indicating a character or area associated with the input medical data;
   a collection unit (213) configured to collect status data and associate it with the correct answer label, the status data being information caused to be displayed on a display by the operator while doing the work of adding the correct answer label, or data indicating an operation performed by the operator; and
   a reliability level outputting unit (215) configured to output a reliability level indicating a probability as to whether or not the correct answer label is correct or not based on the status data,
   the machine learning model comprising:
   a learning unit (216) configured to train a decision making model to which input data (32) is input and which outputs a label, based on the input data, the correct answer label added by the adding unit, and the reliability level associated with the correct answer level and output from the reliability level outputting unit (215),
   the medical information processing apparatus further comprising a label outputting unit configured to output a label indicating a character or area based on the decision making model.

2. The medical information processing apparatus according to claim 1, wherein

   the reliability level outputting unit (215) further comprises a determination unit configured to determine whether or not the correct answer label is correct or not, and
   a reliability level determination model (41) is trained using the status data (42) associated with the correct answer label (43) and a determination result that is output from the determination unit, the reliability level determination model (41) being a machine learning model to which the status data (42) is input and which outputs a reliability level (45) of the correct answer label.

3. The medical information processing apparatus according to claim 2, wherein
   the label and the correct answer label are character information indicating a disease name of a subject associated with the input medical data, or if the input medical data is image data, an area indicating a location of a disease or a specific body part in image data.

4. The medical information processing apparatus according to claim 1, wherein
   the collection unit (213) is configured to collect, as the status data, data relating to an operator's operations, lines of sight, speech, and/or facial expressions that reflect a process of an operator's decision making at the time of doing the work.

5. The medical information processing apparatus according to claim 4, wherein
   the collection unit (213) is configured to collect, as data relating to the line of sight, reference item data which is data relating to an item on which the line of sight of the operator focuses among various items displayed on a display screen for the addition work.

6. The medical information processing apparatus according to claim 5, wherein
   the collection unit is configured to collect as the reference item data, an identifier of a reference item which is an item on which the operator's line of sight focuses.

7. The medical information processing apparatus according to claim 2, wherein
   the collection unit (213) is configured to:

   collect ability data which is data relating to an ability of the operator; and
   train, based on the status data, the ability data and the correct answer label, the reliability level determination model which accepts the status data and the ability data, and outputs the reliability level.

8. The medical information processing apparatus according to claim 7, wherein
   the collection unit (213) is configured to:

   further collect additional data which is data relating to a freshness level, a confidence level, quality, and/or a required time; and
   train, based on the status data, the ability data, the additional data, and the correct answer label, the reliability level determination model which accepts the status data, the ability data, and the additional data, and outputs the reliability level.

9. The medical information processing apparatus according to claim 2, wherein
   the reliability level determination model is a multi-class classification model that outputs the probability of each of multiple classes relating to a result of the decision making as the reliability level.

10. The medical information processing apparatus according to claim 1, wherein

the learning unit is configured to train the decision making model by minimizing a loss function, and
the loss function includes an error between an output of the decision making model and the correct answer label weighted by the reliability level.

11. The medical information processing apparatus according to claim 1, wherein
the reliability level outputting unit (215) is configured to display the reliability level via a display device (25).

12. The medical information processing apparatus according to claim 11, wherein

the label outputting unit is configured to annotate a disease candidate area to a medical image, and
the reliability level outputting unit (215) is configured to display the annotation with a color value according to the reliability level.

13. The medical information processing apparatus according to claim 12, wherein
the correspondence between the reliability level and the color value is set in accordance with a difficulty level of the addition of the annotation.

14. A medical information processing method comprising:

adding, in accordance with an operator's input instruction, a correct answer label used for training a machine learning model to which medical data is input and which outputs a label indicating a character or area used for a medical diagnosis corresponding to the input medical data, the correct answer label indicating a character or area associated with the input medical data;
collecting status data and associate it with the correct answer label, the status data being information caused to be displayed on a display by the operator while doing the work of adding the correct answer label, or data indicating an operation performed by the operator; and
outputting a reliability level indicating a probability as to whether or not the correct answer label is correct or not based on the status data, wherein the machine learning model comprises:
a decision making model to which input data (32) is input and which outputs a label, based on the input data, the correct answer label added by the adding unit, and the outputted reliability level associated with the correct answer level,
the method further comprising outputting a label indicating a character or area based on the decision making model.

FIG. 1

F I G. 2

FIG. 3

34 Reliability level

33 Correct answer label

31 Decision making model

32 Input data

35 Trained decision making model

36 Output data (result of decision making)

32 Input data

42

Status data

41

Reliability level
determination model

43

Correct answer label

42

Status data

44

Trained reliability level
determination model

45

Reliability level

F I G. 4

Start

Obtain examination data ～S1

Display working screen ～S2

Add correct answer label
Collect status data ～S3

S4

Training reliability level
determination model started? NO

YES

Train reliability level determination model
based on status data and correction answer level ～S5

Calculate reliability level from status data using
trained reliability level determination model ～S6

Train examination protocol classification model
based on input data, correct answer label,
and reliability level ～S7

End

F I G. 5

611    612    613    61

| Patient list | Name: Kanja Taro | Protocol A |
| --- | --- | --- |
| Kanja Taro | Order information<br>Age:   ○ 75<br>Sex:   ◎ Male<br>Use of contrast agent: No<br>Examined body parts: Abdomen<br>Diagnosed disease ◎<br>name: HCC | Protocol B |
| Kanja Ichiro | | Protocol C |
| Kanja Hanako | | Protocol D |
| : | | Protocol E |

Confirm

614

⬇

62

| Time and date | User name | Event name | Location of acquisition |
| --- | --- | --- | --- |
| 2021-02-22 16:53:14.864 | Doctor A | Mouse click | Kanja Taro |
| 2021-02-22 16:53:22.432 | Doctor A | Gaze | Age |
| 2021-02-22 16:53:24.356 | Doctor A | Gaze | Use/non-use of contrast agent |
| 2021-02-22 16:53:27.312 | Doctor A | Gaze | Diagnosed disease name |
| 2021-02-22 16:53:43.257 | Doctor A | Mouse click | Protocol B |
| 2021-02-22 16:53:48.012 | Doctor A | Mouse click | Confirmation |

⬇

63

| Determination time | Reference items |
| --- | --- |
| 40 seconds | Patient's name<br>Age<br>Use/non-use of contrast agent<br>Diagnosed disease name |

F I G. 6

| Determination time | Reference items |
|---|---|
| 40 seconds | Patient's name<br>Age<br>Use/non-use of<br>contrast agent<br>Diagnosed<br>disease name |

72

71

Reliability level
determination model
(not trained)

73

| Examination protocol |
|---|
| Protocol A |

F I G. 7

| ID | Determination time | Reference items | Examination protocol |
|---|---|---|---|
| 1 | 60 seconds | ·Patient's name<br>·Age<br>·Sex<br>·Use/non-use of contrast agent<br>·Examined body parts<br>·Diagnosed disease name | Protocol A |
| 2 | 50 seconds | ·Patient's name<br>·Sex<br>·Use/non-use of contrast agent<br>·Examined body parts<br>·Diagnosed disease name | Protocol A |
| 3 | 10 seconds | ·Patient's name | Protocol A |
| 4 | 20 seconds | ·Patient's name<br>·Diagnosed disease name | Protocol B |
| 5 | 23 seconds | ·Patient's name<br>·Diagnosed disease name | Protocol B |

FIG. 8

EP 4 177 904 A1

| Determination time | Reference items |
|---|---|
| 40 seconds | Patient's name<br>Age<br>Use/non-use of<br>contrast agent<br>Diagnosed disease name |

92

91

Reliability level
determination model
(trained)

93

| Protocol A | Protocol B |
|---|---|
| 20% | 80% |

FIG. 9

EP 4 177 904 A1

| ID | Determination time | Reference items | Correct answer label | Reliability level (Protocol A) | Reliability level (Protocol B) |
|---|---|---|---|---|---|
| 1 | 60 seconds | ·Patient's name<br>·Age<br>·Sex<br>·Use/non-use of contrast agent<br>·Examined body parts<br>·Diagnosed disease name | Protocol A | 0.95 | 0.05 |
| 2 | 50 seconds | ·Patient's name<br>·Sex<br>·Use/non-use of contrast agent<br>·Examined body parts<br>·Diagnosed disease name | Protocol A | 0.90 | 0.10 |
| 3 | 10 seconds | ·Patient's name | Protocol A | 0.30 | 0.70 |
| 4 | 20 seconds | ·Patient's name<br>·Diagnosed disease name | Protocol B | 0.10 | 0.90 |
| 5 | 23 seconds | ·Patient's name<br>·Diagnosed disease name | Protocol B | 0.20 | 0.80 |

F I G. 10

EP 4 177 904 A1

| ID | Patient's age | Patient's sex | Diagnosed disease name | Use/non-use of contrast agent |
|---|---|---|---|---|
| 1 | 70 | Male | Hepatoma | Not used |
| 2 | 75 | Male | Hepatoma | Not used |
| 3 | 35 | Male | Hepatoma | Used |
| 4 | 62 | Female | Renal cell carcinoma | Used |
| 5 | 63 | Female | Renal cell carcinoma | Used |

FIG. 11

EP 4 177 904 A1

| | 122 | | |
|---|---|---|---|
| ID | Patient's age | Patient's sex | Diagnosed disease name | Use/non-use of contrast agent |
| 1 | 70 | Male | Hepatoma | Not used |

121 — Examination protocol classification model (not trained)

123 — Protocol A ⊗ 124 — Reliability level (0.95)

F I G. 12

132

| ID | Patient's age | Patient's sex | Diagnosed disease name | Use/non-use of contrast agent |
|----|---------------|---------------|------------------------|-------------------------------|
| 1 | 70 | Male | Hepatoma | Not used |

131

Examination protocol classification model (trained)

133

Protocol A

F I G. 13

EP 4 177 904 A1

| Determination time | Reference items |
|---|---|
| 40 seconds | Patient's name<br>Age<br>Use/non-use of contrast agent<br>Diagnosed disease name |

142

| Examination protocol |
|---|
| Protocol A |

144

141

Reliability level determination model (not trained)

| Ability |
|---|
| Doctor A |

143

F I G. 14

| ID | Operator's name | Determination time | Reference items | Examination protocol |
|---|---|---|---|---|
| 1 | Doctor A | 60 seconds | ·Patient's name<br>·Age<br>·Sex<br>·Use/non-use of<br>  contrast agent<br>·Examined body parts<br>·Diagnosed disease name | Protocol A |
| 2 | Doctor B | 50 seconds | ·Patient's name<br>·Sex<br>·Use/non-use of<br>  contrast agent<br>·Examined body parts<br>·Diagnosed disease name | Protocol B |
| 3 | Doctor A | 10 seconds | ·Patient's name | Protocol A |
| 4 | Doctor B | 20 seconds | ·Patient's name<br>·Diagnosed disease name | Protocol A |
| 5 | Doctor A | 23 seconds | ·Patient's name<br>·Diagnosed disease name | Protocol B |

FIG. 15

EP 4 177 904 A1

161 Reliability level determination model (not trained)

165 Examination protocol — Protocol A

162 
| Determination time | Reference items |
|---|---|
| 40 seconds | Patient's name<br>Age<br>Use/non-use of contrast agent<br>Diagnosed disease name |

163 
| Ability |
|---|
| Doctor A |

164 
| Confidence level | Freshness level |
|---|---|
| 0.9 | 2021-02-22<br>18:43:16.274 |

F I G. 16

F I G. 17

F I G. 18

F I G. 19

Europäisches
Patentamt

European
Patent Office

Office européen
des brevets

**EUROPEAN SEARCH REPORT**

Application Number

EP 22 20 5628

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/160981 A1 (MASUBUCHI NOZOMI [JP] ET AL) 21 May 2020 (2020-05-21) <br> * abstract; figure 1 * <br> * paragraph [0020] - paragraph [0035] * <br> * paragraph [0048] - paragraph [0059] * <br> * paragraph [0076] - paragraph [0082] * <br> ----- | 1-14 | INV. <br> G16H30/40 <br> G16H40/63 <br> G16H50/20 |

TECHNICAL FIELDS
SEARCHED      (IPC)

G16H

The present search report has been drawn up for all claims

| Place of search | Date of completion of the search | Examiner |
|---|---|---|
| Munich | 27 February 2023 | Menschner, Philipp |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 20 5628

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2023

| Patent document cited in search report | | Publication date | Patent family member(s) | | Publication date |
|---|---|---|---|---|---|
| US 2020160981 | A1 | 21-05-2020 | JP | 7201404 B2 | 10-01-2023 |
| | | | JP | 2020086519 A | 04-06-2020 |
| | | | US | 2020160981 A1 | 21-05-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021181131 A